(19) Europäisches Patentamt
European Patent Office
Office européen des brevets

(11) Numéro de publication : **0 577 476 A1**

(12) **DEMANDE DE BREVET EUROPEEN**

(21) Numéro de dépôt : **93401633.8**

(22) Date de dépôt : **25.06.93**

(51) Int. Cl.$^5$ : **C07C 45/28,** C07C 45/36, C07C 47/565, C07C 47/575

(30) Priorité : **29.06.92 FR 9207950**
**26.03.93 FR 9303488**

(43) Date de publication de la demande :
**05.01.94 Bulletin 94/01**

(84) Etats contractants désignés :
**AT BE CH DE DK ES FR GB GR IE IT LI LU MC NL PT SE**

(71) Demandeur : **RHONE-POULENC CHIMIE**
**25, quai Paul Doumer**
**F-92408 Courbevoie Cédex (FR)**

(72) Inventeur : **Costantini, Michel**
**10, rue du Docteur Bonhomme**
**F-69003 Lyon (FR)**
Inventeur : **Laucher, Dominique**
**205, avenue Jean-Jaurès**
**F-69007 Lyon (FR)**
Inventeur : **Fache, Eric**
**118 A, rue Alexis Perroncel**
**F-69100 Villeurbanne (FR)**

(74) Mandataire : **Dutruc-Rosset, Marie-Claude et al**
**RHONE-POULENC CHIMIE Direction de la Propriété Industrielle 25, quai Paul Doumer**
**F-92408 Courbevoie Cédex (FR)**

(54) **Procédé d'oxydation de composés aromatiques porteurs d'un groupe alkyle oxydable.**

(57) La présente invention a pour objet un procédé d'oxydation de composés aromatiques porteurs d'un groupe alkyle oxydable.

Plus précisément, l'invention concerne un procédé d'oxydation de composés aromatiques porteurs d'un groupe alkyle oxydable et d'un groupe hydroxyle et/ou alkoxy caractérisé par le fait qu'il consiste à effectuer la réaction d'oxydation, dans l'eau ou dans un mélange comprenant de l'eau et un solvant organique, en présence d'un catalyseur solide constitué d'une phase active déposée sur support comprenant du palladium éventuellement associé à un autre élément métallique choisi parmi l'étain, le germanium, le tellure et le cuivre.

L'invention concerne plus particulièrement un procédé d'oxydation des crésols en hydroxybenzaldéhydes correspondants.

EP 0 577 476 A1

La présente invention a pour objet un procédé d'oxydation de composés aromatiques porteurs d'un groupe alkyle oxydable.

L'invention vise la préparation d'aldéhydes ou des cétones aromatiques.

Plus particulièrement, l'invention concerne un procédé d'oxydation des composés aromatiques porteurs d'un groupe alkyle oxydable et d'un groupe hydroxyle et/ou alkoxy.

L'invention s'applique donc à la préparation d'aldéhydes ou des cétones hydroxy et/ou alkoxyaromatiques.

Dans sa variante préférée, l'invention se rapporte à un procédé d'oxydation des crésols en hydroxybenzaldéhydes correspondants.

Il est connu selon EP-A 0 012 939, US-A 4 453 016 et US-A 4 471 140, d'oxyder le p-crésol, par l'oxygène moléculaire, en présence d'hydroxyde de sodium ou de potassium et d'un catalyseur tel qu'un composé du cobalt, nickel, chrome ou nickel ce qui conduit aux sels alcalins de p-hydroxybenzaldéhyde.

Une variante préférée consiste à utiliser le méthanol comme solvant réactionnel et de faire appel aux sels de cobalt, à titre de catalyseur.

De tels procédés font appel à un large excès d'hydroxyde de sodium ou de potassium ce qui conduit à des milieux réactionnels très concentrés et visqueux. De plus, pour séparer le p-hydroxybenzaldéhyde formé, il est nécessaire de neutraliser l'excès de base ce qui induit la formation de quantités importantes de sels.

Pour pallier ces inconvénients, on a proposé (US-A 4 748 278) un procédé permettant d'isoler le p-hydroxybenzaldéhyde formé mais cela implique des étapes supplémentaires.

Par ailleurs, il est décrit dans JP-A 63 154 644, un procédé d'oxydation du p-crésol par l'oxygène, en présence d'un catalyseur composé d'acétate de cobalt, de bromure de cobalt et d'acétate de manganèse. La réaction est conduite dans un mélange d'acide acétique et d'anhydride acétique car il est nécessaire de protéger le groupe hydroxyle en le transformant en groupe acétoxy.

Ce procédé présente donc l'inconvénient de nécessiter la protection du groupe hydroxyle. En outre, la réaction n'est pas sélective en aldéhyde car il se forme plus d'acide : le rendement de l'acide acétoxybenzoïque obtenu étant de l'ordre de 80 %.

Un objectif de la présente invention est de fournir un procédé d'oxydation de composés phénoliques, entre autres, de crésols, procédé qui soit conduit en une seule étape, qui n'exige pas la protection du groupe hydroxyle et qui permette d'obtenir l'aldéhyde avec une bonne sélectivité et un bon rendement réactionnel.

Un autre objectif de la présente invention est de disposer d'un procédé permettant également l'oxydation de composés phénoliques O-alkylés porteurs d'un groupe alkyle oxydable.

Il a maintenant été trouvé et c'est ce qui constitue l'objet de la présente invention, un procédé d'oxydation d'un composé aromatique porteur d'un groupe alkyle oxydable de formule générale (I) :

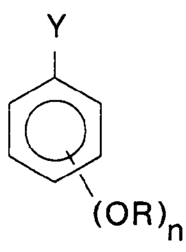

(I)

dans ladite formule (I) :

- Y représente un groupe alkyle oxydable,
- n est un nombre compris entre 1 et 5 , de préférence, égal à 1, 2 ou 3,
- R représente un radical hydrocarboné ayant de 1 à 24 atomes de carbone qui peut être un radical aliphatique saturé, linéaire ou ramifié ; un radical cycloaliphatique saturé, insaturé ou aromatique, monocyclique ou polycyclique; un radical aliphatique saturé, linéaire ou ramifié, porteur d'un substituant cyclique,
- deux groupes R placés sur deux atomes d'oxygène portés par des atomes de carbone vicinaux du noyau aromatique peuvent former ensemble et avec les atomes d'oxygène qui les portent un cycle saturé, insaturé ou aromatique, ayant de 5 à 8 atomes,

caractérisé par le fait qu'il consiste à effectuer la réaction d'oxydation, dans l'eau ou dans un mélange comprenant de l'eau et un solvant organique, en présence d'un catalyseur solide constitué d'une phase active déposée sur support comprenant du palladium éventuellement associé à un autre élément métallique choisi parmi l'étain, le germanium, le tellure et le cuivre.

Dans l'exposé qui suit de la présente invention, on désigne par "composé aromatique à groupe alkyle oxydable", un composé aromatique porteur d'au moins un groupe alkyle oxydable.

Par "composé aromatique", on entend la notion classique d'aromaticité telle que définie dans la littérature, notamment par Jerry MARCH, Advanced Organic Chemistry, 3ème édition, John Wiley and Sons, 1985, p.37 et suivantes.

On entend par "groupe alkyle oxydable", un groupe alkyle dont l'atome de carbone lié directement à l'atome de carbone du noyau aromatique porte au moins deux atomes d'hydrogène libres.

Le substrat aromatique qui est susceptible d'être oxydé dans les conditions de l'invention est un composé aromatique porteur d'un groupe alkyle oxydable et d'un groupe hydroxyle et/ou alkoxy. Le terme "alkoxy" est utilisé dans le présent texte de manière générique, pour désigner tous les radicaux de O-alkylation symbolisés par la formule -O-R; R ayant la signification donnée précédemment.

Le radical R représente donc, un atome d'hydrogène ou un radical hydrocarboné ayant de 1 à 24 atomes de carbone, de préférence un radical "alkyle".

Le radical R peut être de nature quelconque dans la mesure où il reste inerte dans les conditions de la réaction. S'agissant d'un radical aliphatique, celui-ci peut être interrompu par un hétéroatome (par exemple, l'oxygène) ou par un groupe fonctionnel (par exemple, CO) ou porteur d'un substituant (par exemple, un halogène). S'agissant d'un radical cyclique, de préférence cyclohexyle ou phényle, celui-ci peut-être substitué. N'importe quel substituant peut être présent sur ce cycle dans la mesure où il n'interfère pas au niveau du produit désiré. Les substituants peuvent, entre autres, avoir la signification donnée, ci-après, pour le radical $R_4$.

Le substrat aromatique qui est oxydé, conformément au procédé de l'invention, répond plus particulièrement à la formule générale (I') :

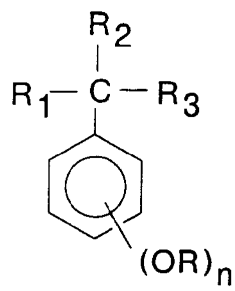

(I')

dans ladite formule (I') :

- n est un nombre égal à 1, 2 ou 3,
- $R_1$ et $R_2$ représentent un atome d'hydrogène,
- $R_3$ représente l'un des groupes suivants :
  . un atome d'hydrogène,
  . un radical alkyle ayant de 1 à 12 atomes de carbone,
  . un radical phényle éventuellement substitué de formule

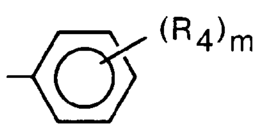

,

  dans lequel $R_4$ représente un atome d'hydrogène, un radical alkyle ayant de 1 à 12 atomes de carbone, un radical alkoxy ayant de 1 à 10 atomes de carbone, un groupe hydroxyle ; m est un nombre égal à 0, 1 , 2 ou 3,
  . un radical alkoxy ayant de 1 à 10 atomes de carbone,
  . un atome d'halogène,
  . un radical du type $R_5$ - CO - X - dans lequel $R_5$ représente un radical alkyle linéaire ou ramifié ayant de 1 à 10 atomes de carbone, un radical phényle, un radical $CF_3$ -, un radical alkoxy ayant de 1 à 10 atomes de carbone ou phénoxy et X représente un lien valentiel ou un atome d'oxygène,
- les radicaux R identiques ou différents, représentent l'un des groupes suivants :
  . un atome d'hydrogène,
  . un radical alkyle ayant de 1 à 12 atomes de carbone, de préférence 1 à 6 atomes de carbone,
  . un radical phényle éventuellement substitué,

- deux groupes R placés sur deux atomes d'oxygène portés par des atomes de carbone vicinaux du noyau aromatique peuvent former ensemble et avec les atomes d'oxygène qui les portent un cycle saturé, insaturé ou aromatique, ayant de 5 à 8 atomes.

Par atome d'halogène, on entend plus particulièrement, un atome de chlore, de brome ou de fluor.

Lorsque n est supérieur ou égal à 1, les groupes R placés sur deux atomes d'oxygène portés par des atomes de carbone vicinaux du noyau aromatique peuvent être liés entre eux pour former un cycle, par un radical alkylène ayant de 1 à 4 atomes de carbone formant ainsi un pont cétalique tel que les radicaux méthylène dioxy ou éthylène dioxy.

La présente invention s'applique tout particulièrement aux composés aromatiques répondant à la formule (I) dans laquelle R représente :

. un atome d'hydrogène,
. un radical alkyle linéaire ou ramifié ayant de 1 à 4 atomes de carbone.

Le procédé de l'invention s'applique tout particulièrement aux composés phénoliques à groupe oxydable qui sont des composés aromatiques à groupe oxydable répondant à la formule générale (I) ou (I') dans laquelle R représente un atome d'hydrogène.

Une classe préférée de substrats susceptibles d'être mis en oeuvre dans le procédé de l'invention sont les substrats phénoliques et l'on peut citer plus particulièrement :

- l'o-crésol
- le p-crésol
- le m-crésol
- l'éthyl-4 phénol
- le (méthoxyméthyl-2) phénol
- le dihydroxy-1,2 méthyl-4 benzène
- le dihydroxy-4,4' diphénylméthane

L'invention est tout à fait adaptée pour effectuer l'oxydation des crésols et plus spécialement pour oxyder le p-crésol en p-hydroxybenzaldéhyde et le m-crésol en m-hydroxybenzaldéhyde.

Comme exemples de substrats aromatiques O-alkylés de formule (I) convenant à la mise en oeuvre de l'invention, on peut mentionner, entre autres :

- l'o-méthylanisole,
- le p-méthylanisole,
- le m-méthylanisole,
- le dioxyméthylène-3,4 toluène,
- le p-phénoxytoluène,
- le p-hydroxyphénoxy-4 toluène.

L'invention est tout à fait adaptée pour effectuer l'oxydation du p-méthylanisole et du dioxyméthylène-3,4 toluène.

Le procédé de l'invention peut être conduit dans un solvant qui peut être de l'eau ou un mélange eau/ solvant organique.

Dans le choix du solvant, il y a lieu de veiller à ce qu'il soit inerte dans les conditions réactionnelles.

Dans l'exposé qui suit de la présente invention, on désigne par "solvant réactionnel", l'eau ou un mélange eau/solvant organique.

L'eau peut être de l'eau ou une solution aqueuse acide, neutre ou légèrement basique.

C'est ainsi que l'on peut envisager une solution aqueuse d'un acide minéral ou d'un sel acide tel que : l'acide borique, l'acide sulfurique, l'acétate de potassium, le carbonate de potassium, l'hydrogénosulfate de sodium, l'hydrogénosulfate de potassium une solution aqueuse d'un sel neutre, par exemple, le nitrate de potassium, le sulfate de potassium, le sulfate de magnésium ; une solution aqueuse d'une base minérale ou d'un sel basique tel que : l'hydroxyde de sodium, l'hydroxyde de potassium, l'hydroxyde d'ammonium, le carbonate basique de potassium.

La concentration de la solution aqueuse en acide, base ou sel varie avantageusement entre $10^{-3}$ et 1 mole/litre.

Conformément au procédé de l'invention, l'eau peut être mise en oeuvre comme solvant réactionnel. Dans ce cas, en l'absence de solvant organique, on fait appel préférentiellement à une solution aqueuse acide, neutre ou légèrement basique telle que précitée.

Convient également à la mise en oeuvre du procédé de l'invention, un mélange eau/ solvant organique.

Par solvant organique, on entend un solvant organique polaire ou apolaire, protique ou aprotique.

Comme exemples de solvants organiques polaires protiques convenant à l'invention, on peut citer, entre autres, les acides carboxyliques ou leurs précurseurs et les alcools ou polyols.

Les acides carboxyliques susceptibles d'être mis en oeuvre peuvent être mono- ou polycarboxyliques. Ce

sont des composés ne présentant pas d'insaturations.

Ils répondent plus particulièrement à la formule générale suivante (II) :

R' - COOH (II)

dans ladite formule (II), R' représente un radical hydrocarboné ayant de 1 à 24 atomes de carbone qui peut être un radical aliphatique acyclique saturé linéaire ou ramifié, un radical cycloaliphatique saturé ou aromatique, monocyclique ou polycyclique.

Ledit radical R' peut porter un autre groupe fonctionnel COOH. Il peut également porter d'autres substituants, par exemple, alkoxy ou halogène dans la mesure où ils n'interfèrent pas dans la réaction.

R' représente plus particulièrement un radical alkyle linéaire ou ramifié ayant de 1 à 4 atomes de carbone, un radical cycloalkyle ayant de 5 à 7 atomes de carbone ou un radical phényle.

Comme exemples d'acides carboxyliques susceptibles d'être mis en oeuvre dans le procédé de l'invention, on peut citer : les acides monocarboxyliques aliphatiques tels que l'acide acétique, l'acide propionique, l'acide butyrique, l'acide valérique, l'acide hexanoïque; les acides dicarboxyliques aliphatiques tels que l'acide malonique, l'acide succinique, l'acide adipique ; les acides cycloaliphatiques tels que l'acide cyclopentane carboxylique, l'acide cyclohexane carboxylique l'acide benzoïque, l'acide naphtoïque, l'acide phénylacétique.

Parmi les acides carboxyliques précités, on fait appel préférentiellement à l'acide acétique ou propionique.

Comme solvants organiques polaires protiques qui conviennent à la présente invention, on peut également avoir recours aux alcools mono- ou polyhydroxylés.

On choisit plus particulièrement les alcools répondant à la formule générale (III) :

R" - OH (III)

dans ladite formule (III), R" représente un radical hydrocarboné ayant de 1 à 24 atomes de carbone qui peut être un radical aliphatique acyclique saturé linéaire ou ramifié ou un radical cycloaliphatique saturé, monocyclique.

Ledit radical R" peut porter un autre groupe fonctionnel OH. Il peut également porter d'autres substituants, par exemple, alkoxy ou halogène dans la mesure où ils n'interfèrent pas dans la réaction.

R" représente plus particulièrement un radical alkyle linéaire ou ramifié ayant de 1 à 4 atomes de carbone, un radical cycloalkyle ayant de 5 à 7 atomes de carbone.

Comme exemples d'alcools mono- ou polyhydroxylés susceptibles d'être mis en oeuvre dans le procédé de l'invention, on peut citer : les monoalcools aliphatiques tels que le méthanol, l'éthanol, le propanol, le butanol, le sec-butanol, le tert-butanol, le pentanol, l'hexanol; les dialcools aliphatiques tels que l'éthylène glycol, le diéthylène glycol, le propylène glycol les alcools cycloaliphatiques tels que le cyclopentanol, le cyclohexanol.

Parmi les différents alcools précités, les alcools préférés sont le méthanol, l'éthanol, le tert-butanol.

Conviennent également à la mise en oeuvre du procédé de l'invention, des solvants organiques polaires aprotiques et l'on peut citer plus particulièrement :

- les composés nitrés tels que le nitrométhane, le nitroéthane, le nitro-1 propane, le nitro-2 propane ou leurs mélanges, le nitrobenzène,
- les nitriles aliphatiques ou aromatiques comme l'acétonitrile, le propionitrile, le butanenitrile, l'isobutanenitrile, le benzonitrile, le cyanure de benzyle,
- la tétraméthylène sulfone (sulfolane).

Parmi les solvants précités, on met en oeuvre, préférentiellement, l'acétonitrile.

On peut aussi mettre en oeuvre dans le procédé de l'invention, des solvants aprotiques peu polaires tels que notamment :

- les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,
- les esters neutres phosphoriques tels que, notamment, le phosphate de triméthyle, le phosphate de triéthyle, le phosphate de tributyle, le phosphate de triisobutyle, le phosphate de tripentyle,
- le carbonate d'éthylène,
- les hydrocarbures halogénés aliphatiques ou aromatiques, et plus particulièrement, les hydrocarbures perchlorés tels que notamment le tétrachlorure de carbone, le tétrachloroéthylène, l'hexachloroéthane ; les hydrocarbures partiellement chlorés tels que le dichlorométhane, le chloroforme, le dichloro-1,2 éthane, le trichloro-1,1,1 éthane, le tétrachloro-1,1,2,2 éthane, le pentachloroéthane, le trichloroéthylène, le chloro-1 butane, le dichloro-1,2 butane ; le monochlorobenzène, le dichloro-1,2 benzène, le dichloro-1,3 benzène, le dichloro-1,4 benzène , le trichloro-1,2,4 benzène ou des mélanges de différents chlorobenzènes ; le bromoforme, le bromoéthane ou le dibromo-1 ,2 éthane ; le monobromobenzène ou des mélanges de monobromobenzène avec un ou plusieurs dibromobenzènes ; le bromo-1 naphtalène.

On utilise préférentiellement, le dioxane.

Une autre classe de solvants convenant également à l'invention, sont les solvants aprotiques non polaires tels que par exemple, les hydrocarbures aliphatiques ou aromatiques et l'on peut citer plus particulièrement l'hexane, l'heptane, l'octane, le nonane, le décane ou le cyclohexane, le benzène et le naphtalène.

Selon le procédé de l'invention, l'oxydation du substrat aromatique peut être conduite dans un mélange eau/solvant organique.

La quantité d'eau dans le mélange eau/solvant organique peut varier largement. Ainsi, le rapport pondéral entre l'eau et le solvant organique peut varier entre 0,05 et 0,95, et de préférence entre 0,20 et 0,80.

L'une des caractéristiques du procédé de l'invention est qu'il y a toujours de l'eau dans le mélange réactionnel.

Le rapport molaire eau/substrat aromatique à oxyder peut varier entre 20 et 1000 et se situe de préférence aux environs de 100.

La concentration du substrat aromatique à oxyder, dans le solvant réactionnel peut varier dans de larges limites. Ainsi, elle peut être comprise entre 0,01 mole et 5 moles de substrat aromatique par litre de solvant réactionnel et de préférence de 0,05 à 1,0 mole par litre.

Selon la présente invention, on met en oeuvre un catalyseur solide qui comprend au moins l'élément métallique palladium, celui-ci pouvant être associé à d'autres éléments métalliques choisis parmi l'étain, le germanium, le tellure et le cuivre. On peut donc faire appel à des catalyseurs bi- ou tri-métalliques.

Comme exemples de catalyseurs convenant bien à la présente invention, on peut citer les catalyseurs comprenant les éléments suivants : palladium, palladium-étain, palladium-germanium, palladium-cuivre, palladium-tellure, palladium-tellure-cuivre, palladium-étain-cuivre.

On a recours avantageusement aux catalyseurs à base de palladium-étain, palladium-germanium.

Les éléments métalliques précités sont mis en oeuvre sur un support dont la nature peut être variable. C'est ainsi que peuvent convenir à la présente invention, notamment, les charbons actifs, les gels de silice, les mélanges silice-alumine, l'alumine, les argiles, la bauxite, la magnésie et la terre de diatomées.

Pour une bonne mise en oeuvre du procédé de l'invention, on choisit à titre de supports préférentiels, les charbons généralement activés par un traitement bien connu effectué à l'acide nitrique.

La phase active constituée de palladium éventuellement additionnée d'un autre élément métallique tel que précité représente généralement au plus 20 % du poids du catalyseur solide bien que des teneurs plus élevées puissent être utilisées.

En ce qui concerne la teneur minimale, la phase active représente au moins 0,1 % du poids du catalyseur solide.

La quantité de phase active dans le catalyseur peut varier largement, elle représente de préférence de 0,5 à 10 % du poids du catalyseur solide.

Lorsque le catalyseur présente des éléments métalliques additionnels, la quantité de ceux-ci est telle que le rapport molaire entre le palladium et les autres éléments métalliques est compris, de préférence entre 0,01 et 10, et encore plus préférentiellement entre 0,1 et 5,0.

La quantité de catalyseur à mettre en oeuvre par rapport au substrat aromatique à oxyder peut varier dans de larges limites. Ainsi, la quantité de catalyseur exprimée par le rapport molaire entre le ou les éléments métalliques et le composé aromatique porteur d'un groupe alkyle oxydable peut varier entre 0,01 et 0,001, de préférence entre 0,05 et 0,01.

Pour préparer le catalyseur supporté utile à la mise en oeuvre du procédé de la présente invention, on peut recourir à des techniques classiques, connues en elles-mêmes, de préparation de catalyseurs métalliques supportés. On peut se référer, notamment, pour la préparation des différents catalyseurs à l'ouvrage :
[J. F. LEPAGE "Catalyse de contact", conception, préparation et mise en oeuvre des catalyseurs industriels, Edition Technip (1978)].

Le catalyseur peut être préparé, par exemple, en introduisant un support dans une solution que l'on prépare en dissolvant au moins un composé approprié du (ou des) éléments(s) choisi(s) le dépôt du ou des éléments actifs sur le support est réalisé en distillant le solvant et la masse de contact ainsi obtenue est soumise à une réduction au moyen d'un courant d'hydrogène ou par un composé réducteur tel que l'hydrazine, le méthanol ou le formaldéhyde.

Selon un autre mode de préparation classique, le dépôt du ou des composés apportant les éléments actifs sur le support est réalisé en précipitant les composés de manière en soi connue et en soumettant la masse de contact ainsi obtenue à une réduction au moyen, notamment, de l'hydrogène, de l'hydrazine, du méthanol ou du formaldéhyde.

D'autres modes de préparation sont également possibles, en particulier l'imprégnation d'un support au moyen d'une solution du ou des composés appropriés, en présence d'un réducteur organique tel que précité.

Le dépôt sur le support de plusieurs éléments métalliques peut bien entendu être réalisé simultanément ou successivement.

Généralement, il est préférable de faire appel à un catalyseur pré-réduit c'est-à-dire soumis à une réduction telle que précisée ci-dessus. La réduction du catalyseur s'effectue à chaud, le plus souvent à une température comprise entre 200°C et 250°C. Elle est particulièrement avantageuse dans le cas où l'on fait appel à des catalyseurs ne comprenant que du palladium.

La nature des composés apportant les éléments métalliques utilisés pour la préparation des catalyseurs de l'invention n'est pas critique.

On peut faire appel aux métaux eux-mêmes tels que le palladium, l'étain, le germanium, le tellure et le cuivre.

A titre d'exemples de composés susceptibles d'être mis en oeuvre pour la préparation des catalyseurs de l'invention, on peut citer notamment : l'oxyde de palladium hydraté ou non, le dioxyde de palladium, le nitrate de palladium, l'acétate de palladium. Pour ce qui est des éléments métalliques additionnels, on peut utiliser, entre autres, les oxydes, nitrates, carboxylates, alcoolates d'étain, de germanium, de tellure ou de cuivre ou des composés organométalliques dans lesquels les métaux précités sont liés à un atome d'hydrogène et/ou des radicaux alkyle ayant de préférence de 1 à 4 atomes de carbone. Les sels préférés sont les suivants : les composés de l'étain II tels que l'acétate d'étain II, l'octoate d'étain II, l'éthylhexanoate d'étain ; le monoxyde ou le trioxyde de tellure ; l'oxyde de germanium, l'éthylate de germanium, le tétrabutylgermanium ; l'oxyde cuivreux, l'oxyde cuivrique, le sulfate cuivrique, l'acétate cuivrique, le méthylate cuivrique.

La taille des particules de catalyseur se situe généralement entre 0,1 et 1,0 mm, étant bien entendu que des dimensions plus grandes ou plus petites peuvent être également retenues. La taille des particules telle que précitée dépend du mode opératoire choisi.

Comme mentionné précédemment, les composés aromatiques porteurs d'un groupe alkyle oxydable sont oxydés dans un solvant réactionnel tel que défini en présence d'un catalyseur solide défini ci-avant et d'un agent oxydant.

La réaction est avantageusement conduite en phase liquide, en mettant en suspension, le catalyseur solide dans le milieu réactionnel comprenant le composé aromatique porteur d'un groupe alkyle oxydable, le solvant réactionnel et un agent oxydant.

Comme agents oxydants susceptibles d'être mis en oeuvre dans le procédé de l'invention, on peut citer notamment, le peroxyde d'hydrogène, l'acide peracétique, l'hydroperoxyde de tertiobutyle, l'hydroperoxyde de cyclohexyle.

Le peroxyde d'hydrogène est mis en oeuvre avantageusement sous sa forme commerciale à savoir une solution aqueuse dont la concentration se situe aux environs de 70 %.

La quantité d'agent oxydant introduite peut varier largement. Généralement, elle est égale à la quantité stoechiométrique voire-même en léger excès de 20 % par rapport à la quantité stoechiométrique.

A titre d'agents oxydants, on fait appel préférentiellement à l'oxygène moléculaire ou un gaz en contenant.

Ce gaz peut être de l'oxygène pur ou de l'oxygène dilué avec un gaz inerte, par exemple l'azote ou un gaz rare, de préférence, l'argon. On peut donc faire appel à l'air.

La quantité d'oxygène à mettre en oeuvre n'est pas critique dans la mesure où elle est telle que ni les gaz d'alimentation, ni une éventuelle phase gazeuse susceptible d'apparaître dans la zone réactionnelle se trouve dans la plage des compositions explosives, compte-tenu des autres paramètres ou conditions réactionnelles choisies. La quantité d'oxygène peut être en excès ou en défaut par rapport à la stoechiométrie de la réaction, vis-à-vis du substrat à oxyder.

La pression de réaction varie entre la pression atmosphérique et environ 200 bar. On préfère une pression entre 1 et 100 bar.

Le procédé de l'invention peut être conduit sous pression atmosphérique, en faisant buller de l'oxygène pur, à un débit, par exemple, de 1 à 50 litres/heure.

Une autre variante de l'invention consiste à travailler sous pression, en autoclave. Dans ce cas, on met en oeuvre de l'oxygène dilué, de préférence de l'air, sous une pression avantageusement de l'ordre de 80 à 100 bar.

La température de la réaction est généralement comprise entre 40°C et 190°C, et de préférence entre 50°C et 120°C.

D'un point de vue pratique, on charge dans le réacteur dans un ordre quelconque : le solvant réactionnel, le catalyseur solide et le substrat aromatique à oxyder.

Dans le cas où l'agent oxydant est sous forme liquide, il est introduit en même temps que les autres réactifs.

On agite le milieu réactionnel et on le chauffe à la température réactionnelle choisie. Lorsque l'agent oxydant est sous forme gazeuse, on envoie alors l'oxygène moléculaire ou un gaz en contenant.

En fin de réaction, on sépare le catalyseur solide par les techniques classiques de séparation solide/liquide,

de préférence par filtration ou par décantation. On récupère le composé oxydé obtenu, aldéhyde ou cétone, par tout moyen approprié, par exemple, par distillation du solvant réactionnel.

Comme mentionné précédemment, le procédé de l'invention s'applique tout particulièrement à l'oxydation des crésols, de préférence, le p-crésol en hydroxybenzaldéhydes.

On ne sortira pas du cadre de la présente invention, à mettre en oeuvre le procédé de l'invention sur un composé aromatique à groupe oxydable répondant à la formule (I) dans laquelle le noyau benzénique est remplacé par un noyau naphténique.

Les exemples qui suivent, illustrent l'invention sans toutefois la limiter.

Dans les exemples 1 à 23, l'appareillage utilisé est toujours le même. Il s'agit d'un ballon en verre de 100 ml, muni d'une agitation centrale efficace, d'un réfrigérant, d'une alimentation d'oxygène par un tube plongeant. Le chauffage est assuré par un bain d'huile.

Dans les exemples, les abréviations ont la signification suivante :

$$\text{Taux de conversion} = \frac{\text{nombre de moles de substrat aromatique transformées}}{\text{nombre de moles de substrat aromatique introduites}}$$

$$\text{Sélectivité} = \frac{\text{nombre de moles d'aldéhyde formées}}{\text{nombre de moles de substrat aromatique transformées}}$$

Les pourcentages donnés dans les exemples sont exprimés en poids.

Exemple n° 1

a) Traitement du noir de carbone servant de support

Du noir de carbone activé fourni par la Société CECA S.A. de type 3S et de surface BET de 1150 $m^2/g$ (100 g) est mis en suspension dans une solution aqueuse d'acide nitrique à 30 % (600 ml). Le mélange est porté à reflux pendant 2 heures. La solution aqueuse est éliminée par filtration et le noir de carbone est lavé à l'eau déminéralisée jusqu'à obtention d'un pH des eaux de lavage égal à 4. Le noir de carbone est séché à 90°C, sous pression réduite de 20 mm de mercure.

b) Préparation du catalyseur contenant du palladium et du germanium

Dans un autoclave de 300 ml, on charge du noir de carbone (10 g), ainsi traité, en suspension dans du toluène (150 ml), puis on charge du tétrabutylgermanium (6,02 g, 20 mmol) et de l'acétate de palladium (1,08 g, 4,42 mmol).

On chauffe à 80°C, pendant 2 heures, avec agitation efficace sous 20 $kg/cm^2$ d'hydrogène.

L'autoclave est refroidi, le catalyseur récupéré par filtration sous argon et séché sous pression réduite de 20 mm de mercure.

c) Oxydation du p-crésol

Le catalyseur (1 g) ainsi préparé a été mis en réaction suivant le mode opératoire donné ci-après.

On charge dans le réacteur :

- 10 ml d'acide acétique,
- 40 ml d'eau,
- 0,98 g (10 mmol) d'acétate de potassium,
- 1,1 g (10 mmol) de p-crésol,
- 1 g de catalyseur tel que préparé palladium/germanuim sur carbone.

Il est précisé et ceci est valable pour tous les exemples que l'acide acétique mis en oeuvre est de l'acide acétique à 99 %.

On agite, chauffe à 100°C pendant deux heures, en admettant de l'oxygène à raison de 5 litres/heure.

Le réacteur est refroidi et le contenu dosé par chromatographie liquide haute performance (CLHP).

La conversion du p-crésol est de 50 %.

On obtient 0,8 mmole de p-(hydroxyméthyl) phénol, 2,2 mmol de p-hydroxybenzaldéhyde et 0,3 mmole d'acide p-hydroxybenzoïque.

Exemple n° 2

Dans cet exemple, on met en oeuvre comme catalyseur, du palladium déposé sur noir de carbone.

Le catalyseur a été obtenu par précipitation du palladium, à partir du chlorure de palladium selon une technique bien connue décrite dans l'ouvrage : [J. F. LEPAGE "Catalyse de contact", conception, préparation et mise en oeuvre des catalyseurs industriels, Edition Technip (1978)].

On charge dans le réacteur :

- 10 ml d'acide acétique,
- 40 ml d'eau,
- 0,98 g (10 mmol) d'acétate de potassium,
- 1,1 g (10 mmol) de p-crésol,
- 1 g de catalyseur à savoir du palladium sur noir de carbone (3S) comprenant 5 % en poids de palladium.

On agite, chauffe à 100°C pendant deux heures, en admettant de l'oxygène à raison de 5 litres/heure.

Le réacteur est refroidi et le contenu dosé par chromatographie liquide haute performance (CLHP).

La conversion du p-crésol est de 60 %.

On obtient 0,18 mmole de p-(acétoxyméthyl)phénol, 1,11 mmol de p-hydroxybenzaldéhyde et 0,2 mmole d'acide p-hydroxybenzoïque.

Exemple n° 3

a) Préparation du catalyseur contenant du palladium et de l'étain.

Dans un ballon tel que précédemment décrit, on charge :

- 400 ml d'acide acétique,
- 2,44g (10 mmol) d'acétate de palladium Pd $(OAc)_2$,
- 9,8 g (100 mmol) d'acétate de potassium,
- 20 g de noir de carbone traité selon le mode opératoire de l'exemple n° 1.

On agite 15 minutes à température ambiante puis on additionne 16,1 g (40 mmol) d'éthylhexanoate d'étain II

On chauffe 4 heures à 110°C sous agitation.

Le ballon est refroidi, le catalyseur recueilli par filtration sous argon, lavé avec 2 fois 50 ml d'acide acétique et séché à 50°C sous 20 mm de mercure. Le catalyseur renferme 4,3 % en poids de palladium, et 2 % en poids d'étain.

b) Oxydation du p-crésol

Le catalyseur (1,0 g) ainsi préparé est mis en réaction suivant le mode opératoire utilisé pour l'exemple n° 2.

La conversion du p-crésol et de 96 %.

On obtient 0,3 mmole de p-(hydroxyméthyl)-phénol, 5,2 mmol de p-hydroxybenzaldéhyde, 1 mmole d'acide p-hydroxybenzoïque.

Exemple n° 4

Le catalyseur (0,5 g) préparé dans l'exemple n° 3 est mis en réaction suivant le mode opératoire utilisé pour l'exemple n° 2 sauf que la réaction est effectuée dans un mélange de 30 ml d'acide acétique et de 20 ml d'eau.

La température de réaction est de 100°C.

La conversion du p-crésol est de 87 %.

On obtient 0,9 mmole de p-(acétoxyméthyl)phénol, 5,6 mmol de p-hydroxybenzaldéhyde et 0,75 mmole d'acide p-hydroxybenzoïque.

Exemple n° 5

Le catalyseur (0,5 g) préparé dans l'exemple n° 3 est mis en réaction suivant le mode opératoire utilisé pour l'exemple n° 2 sauf que la réaction est effectuée dans un mélange de 20 ml de méthanol et de 30 ml d'une solution aqueuse d'acide sulfurique à pH = 1.

La température de réaction est de 80°C.

La conversion du p-crésol est de 80 %.

On obtient 5,4 mmol de p-hydroxybenzaldéhyde et 0,7 mmole de p-hydroxybenzoate de méthyle.

Exemple n° 6

Le catalyseur (1 g) préparé dans l'exemple n° 3 est mis en réaction suivant le mode opératoire utilisé pour l'exemple n° 2, sauf que l'on remplace l'acide acétique par du tertiobutanol et que la température de réaction

est de 80°C.

La conversion du p-crésol est de 50 %.

On obtient 2,2 mmol de p-hydroxybenzaldéhyde et 0,2 mmole d'acide p-hydroxybenzoïque.

Exemple n° 7

Dans cet exemple, on met en oeuvre un catalyseur palladium/cuivre/étain préparé à partir d'un catalyseur palladium/cuivre sur carbone fabriqué par Engelhard contenant 53 % en poids de palladium, 1,5 % en poids de cuivre et 53 % en poids d'eau.

On charge :
- 2 g d'un catalyseur palladium/cuivre sur carbone,
- 20 ml d'acide acétique,
- 30 ml d'eau,
- 1,02 g (9,4 mmol) de p-crésol,
- 0,9 g (2,25 mmol) d'éthylhexanoate d'étain II.

On chauffe 2 heures à 100°C en agitant et en admettant de l'oxygène à raison de 5 litres/heure.

La conversion du p-crésol est de 70 %.

On obtient 2,9 mmol de p-hydroxybenzaldéhyde et 0,8 mmole d'acide p-hydroxybenzoïque.

Exemple n° 8

a) Préparation du catalyseur contenant du palladium. du tellure et du cuivre

On dissout dans une solution aqueuse à 30 % d'acide nitrique :
- 1,63 g (7,3 mmol) d'acétate de palladium, $Pd(OAc)_2$,
- 0,105 g (0,66 mmol) de dioxyde de tellure $TeO_2$,
- 4 g (20 mmol) d'acétate de cuivre $Cu(OAc)_2,H_2O$.

On met en suspension dans cette solution du noir de carbone (15 g) traité selon l'exemple n° 1. On évapore le solvant sous pression réduite et le solide ainsi obtenu est séché à 80°C sous pression réduite de 20 mm de mercure.

Le catalyseur est réduit pendant 2 heures à 200°C et 2 heures à 400°C par un courant d'azote (60 litres/heure) saturé en méthanol.

Puis il est traité pendant 15 heures à 300°C avec de l'azote (60 litres/heure) contenant 2 % d'oxygène et à nouveau réduit pendant 2 heures à 200°C et 2 heures à 400°C avec un courant d'azote saturé en méthanol.

b) Oxydation du p-crésol

Un essai a été réalisé avec le catalyseur (1 g) ainsi préparé.

On charge dans le réacteur :
- 1 g de catalyseur,
- 20 ml d'acide acétique,
- 30 ml d'eau,
- 1,22 g (11,3 mmol) de p-crésol.

On chauffe 2 heures à 100°C en agitant et en admettant de l'oxygène à raison de 5 litres/heure.

La conversion du p-crésol est de 50 %.

On obtient 2,9 mmol de p-hydroxybenzaldéhyde et 0,3 mmole d'acide p-hydroxybenzoïque.

Exemple n° 9

Le catalyseur (0,5 g) préparé dans l'exemple n° 3 est mis en réaction suivant le mode opératoire utilisé pour l'exemple n° 2 sauf que la réaction est effectuée dans un mélange de 25 ml d'eau et de 25 ml de dioxane.

La température de réaction est de 90°C.

La conversion du p-crésol est de 60 %.

On obtient 1,2 mmol de p-(hydroxyméthyl)phénol et 3,3 mmol de p-hydroxybenzaldéhyde.

Exemple n° 10

Dans cet exemple, on conduit la réaction en milieu biphasique.

Le catalyseur (0,5 g) préparé dans l'exemple n° 3 est mis en réaction suivant le mode opératoire utilisé pour l'exemple n° 2 sauf que la réaction est effectuée dans un mélange de 25 ml de chlorobenzène, 12,5 ml

d'acide acétique et 12,5 ml d'eau.

La conversion du p-crésol est de 35 %.

On obtient 1,6 mmol de p-(hydroxyméthyl)phénol et 1,0 mmole de p-hydroxybenzaldéhyde.

Exemple n° 11

Dans cet exemple, on conduit la réaction en utilisant le peroxyde d'hydrogène comme oxydant.

Le catalyseur (1,0 g) préparé dans l'exemple n° 3 est chargé dans le réacteur ainsi que :

- 25 ml d'eau,
- 25 ml d'acide acétique,
- 25 mmol de peroxyde d'hydrogène
- 10 mmol de p-crésol,
- 10 mmol d'acétate de potassium.

On agite et l'on chauffe 2 heures à 100°C.

La conversion du peroxyde d'hydrogène est de 100% et celle du p-crésol de 84%.

On obtient 3,0 mmol de p-hydroxybenzaldéhyde et 2,7 mmol d'acide p-hydroxybenzoïque.

Exemple n° 12

Dans cet exemple, on conduit la réaction sous pression.

Le catalyseur (0,5 g) préparé dans l'exemple n° 3 est chargé dans un autoclave de 150 ml ainsi que :

- 25 ml d'eau,
- 25 ml d'acide acétique,
- 10 mmol de p-crésol,
- 10 mmol d'acétate de potassium.

L'autoclave es mis sous pression de 40 bar d'air contenant en volume, 20 % d'oxygène et 80 % d'azote et chauffé à 130°C pendant 2 heures.

L'autoclave est refroidi et ramené à la pression atmosphérique.

La conversion du p-crésol est de 100 %.

On obtient 6,8 mmol de p-hydroxybenzaldéhyde et 1 mmole d'acide p-hydroxybenzoïque.

Exemple n° 13

Le catalyseur utilisé est le catalyseur contenant du palladium et de l'étain de l'exemple n° 3.

On charge dans le réacteur :

- 25 ml d'acide acétique,
- 25 ml d'eau,
- 0,98 g (10 mmol) d'acétate de potassium,
- 1,1 g (10 mmol) de p-crésol,
- 1 g de catalyseur.

On agite et chauffe à 100°C pendant deux heures, en admettant de l'oxygène à raison de 5 litres/heure.

Le réacteur est refroidi et le contenu dosé par chromatographie liquide haute performance (CLHP).

La conversion du p-crésol est de 79 %.

On obtient 0,84 mmol de p-(acétoxyméthyl)phénol, 0,28 mmol de p-(hydroxyméthyl)phénol, 4,82 mmol de p-hydroxybenzaldéhyde, 0,59 mmol d'acide p-hydroxybenzoïque.

Exemple n° 14

Dans cet exemple, on met en oeuvre comme catalyseur, du palladium déposé sur noir de carbone.

Le catalyseur a été obtenu par précipitation du palladium, à partir du chlorure de palladium selon la technique décrite par J. F. LEPAGE (loc. cit.).

On charge dans le réacteur :

- 25 ml d'acide acétique,
- 25 ml d'eau,
- 0,98 g (10 mmol) d'acétate de potassium,
- 1,1 g (10 mmol) de p-crésol,
- 0,5 g de catalyseur à savoir du palladium sur noir de carbone (3S) comprenant 3 % en poids de palladium.

On agite, chauffe à 100°C pendant quatre heures, en admettant de l'oxygène à raison de 5 litres/heure.

Le réacteur est refroidi et le contenu dosé par chromatographie liquide haute performance (CLHP).
La conversion du p-crésol est de 11 %.
On obtient 0,26 mmol de p-(acétoxyméthyl)phénol, 0,37 mmol de p-(hydroxyméthyl)phénol, 0,21 mmol de p-hydroxybenzaldéhyde et 0,2 mmol d'acide p-hydroxybenzoïque.

Exemple n° 15

Dans cet exemple, on met en oeuvre le catalyseur de l'exemple 14.
Après traitement à 200°C sous courant d'hydrogène de 1 litre/heure, le catalyseur est mis en réaction suivant le mode opératoire de l'exemple 14 à l'exception que la durée de la réaction est de deux heures au lieu de quatre.
La conversion du p-crésol est de 40 %.
On obtient 1,2 mmol de p-(acétoxyméthyl)phénol, 1,0 mmol de p-(hydroxyméthyl)phénol, 2,1 mmol de p-hydroxybenzaldéhyde et 0,2 mmol d'acide p-hydroxybenzoïque.

Exemple n° 16

Dans cet exemple, on met en oeuvre le catalyseur de l'exemple 14 suivant le mode opératoire de l'exemple 14 à l'exception que la durée de la réaction est de quatre heures trente minutes.
La conversion du p-crésol est de 87 %.
On obtient 1,8 mmol de p-(acétoxyméthyl)phénol, 6,0 mmol de p-hydroxybenzaldéhyde et 0,7 mmol d'acide p-hydroxybenzoïque.

Exemple n° 17

Dans cet exemple, on met en oeuvre le catalyseur de l'exemple 14 suivant le mode opératoire de l'exemple 14 à l'exception que la durée de la réaction est de 21 heures.
La conversion du p-crésol est de 100 %.
On obtient 6,9 mmol de p-hydroxybenzaldéhyde et 3,0 mmol d'acide p-hydroxybenzoïque.

Exemple n° 18

Dans cet exemple, on met en oeuvre comme catalyseur du palladium déposé sur silice SPHEROSIL X400LS®. Le palladium est déposé sur le support selon la technique classique d'échange en utilisant comme précurseur de palladium, le composé $Pd(NH_3)_4(OH)_2$.
Le catalyseur est pré-réduit comme décrit dans l'exemple 15 puis l'on charge dans le réacteur :
- 25 ml d'acide acétique,
- 25 ml d'eau,
- 0,98 g (10 mmol) d'acétate de potassium,
- 1,1 g (10 mmol) de p-crésol,
- 0,5 g de catalyseur à savoir du palladium sur silice SPHEROSIL X400LS comprenant 3 % en poids de palladium.

On agite, chauffe à 100°C pendant deux heures, en admettant de l'oxygène à raison de 5 litres/heure.
Le réacteur est refroidi et le contenu dosé par chromatographie liquide haute performance (CLHP).
La conversion du p-crésol est de 38 %.
On obtient 0,6 mmol de p-(acétoxyméthyl)phénol, 0,5 mmol de p-(hydroxyméthyl)phénol, 2,3 mmol de p-hydroxybenzaldéhyde et 0,2 mmol d'acide p-hydroxybenzoïque.

Exemples n° 19a à 19d

Dans cet exemple, on met en oeuvre comme catalyseur, du palladium sur noir de carbone.
Dans un premier essai, on fait appel à un catalyseur neuf puis dans les essais suivants, on met en oeuvre un catalyseur recyclé.
Le catalyseur est pré-réduit comme décrit dans l'exemple 15 puis l'on charge dans le réacteur :
- 25 ml d'acide acétique,
- 25 ml d'eau,
- 0,98 g (10 mmol) d'acétate de potassium,
- 1,1 g (10 mmol) de p-crésol,

- 0,5 g de catalyseur à savoir du palladium sur noir de carbone (3S) comprenant 3 % en poids de palladium.

On agite, chauffe à 100°C pendant deux heures, en admettant de l'oxygène à raison de 5 litres/heure.

Le réacteur est refroidi et le milieu réactionnel filtré.

Le catalyseur est lavé à l'acide acétique.

Le filtrat et les eaux de lavage sont dosés par CLHP et le catalyseur utilisé pour une nouvelle réaction d'oxydation.

Les résultats obtenus sont consignés dans le tableau (I).

Tableau (I)

| Ref. | Nombre de recyclage | Taux de conversion | p-(hydroxy méthyl) phénol mmol | p-(acétoxy méthyl) phénol mmol | p-hydroxy benzaldéhyde mmol | p-hydroxy benzoïque mmol |
|---|---|---|---|---|---|---|
| 19a | 0 | 56,2 | 0,82 | 1,25 | 2,84 | 0,31 |
| 19b | 1 | 63,7 | 0,78 | 1,03 | 3,66 | 0,31 |
| 19c | 2 | 65,8 | 0,55 | 0,79 | 4,97 | 0,38 |
| 19d | 3 | 75,8 | 0,20 | 0,58 | 5,21 | 0,81 |

Exemple comparatif A

Le catalyseur utilisé est le catalyseur contenant du palladium et de l'étain de l'exemple n° 3.

On reproduit l'exemple 13 à la différence que le milieu réactionnel ne comporte pas d'eau. On met en oeuvre 50 ml d'acide acétique.

On agite et chauffe à 100°C pendant deux heures, en admettant de l'oxygène à raison de 5 litres/heure.

Le réacteur est refroidi et le contenu dosé par chromatographie liquide haute performance (CLHP).

La conversion du p-crésol est de 100 %.

On obtient 5,2 mmol de p-(acétoxyméthyl)phénol, 2,4 mmol de p-hydroxybenzaldéhyde et 1,2 mmol d'acide p-hydroxybenzoïque.

Exemple n° 20

a) Préparation du catalvseur contenant du palladium déposé sur noir de carbone

Le catalyseur a été obtenu par précipitation du palladium, à partir du chlorure de palladium selon la technique décrite par J. F. LEPAGE (loc. cit.).

La teneur du palladium dans le catalyseur est de 3%.

Le catalyseur est réduit à 200°C sous courant d'hydrogène (1litre/heure) pendant deux heures.

b) Oxydation du méthyl-4 anisole

Dans un réacteur de 300ml, on charge :

- 25 ml d'acide acétique,
- 25 ml d'eau,
- 0,98 g (10 mmol) d'acétate de potassium,
- 1,2g (10 mmol) de méthyl-4 anisole,
- 1g de catalyseur tel que préparé palladium/noir de carbone.

Il est précisé et ceci est valable pour tous les exemples que l'acide acétique mis en oeuvre est de l'acide acétique à 99 %.

On agite, chauffe à 100°C pendant quatre heures, en admettant de l'oxygène à raison de 5 litres/heure.

Le réacteur est refroidi et le contenu dosé par chromatographie liquide haute performance (CLHP).

La conversion du méthyl-4 anisole est de 60%.

On obtient 0,16 mmol de p-(hydroxyméthyl)anisole, 2,6 mmol de p-(acétoxyméthyl)anisole, 1,9 mmol de p-méthoxybenzaldéhyde et 0,4 mmol d'acide p-méthoxybenzoïque.

Exemple n° 21

On reproduit l'exemple 20 à la différence près que l'on remplace le méthyl-4 anisole par le méthyl-2 anisole.

La conversion du méthyl-2 anisole est de 50%.

On obtient 0,2 mmol d'o-(hydroxyméthyl)anisole, 2,7 mmol d'o-(acétoxyméthyl)anisole, 0,52 mmol d'o-méthoxybenzaldéhyde et 0,06 mmol d'acide o-méthoxybenzoïque.

Exemple n° 22

On reproduit l'exemple 20 à la différence près que l'on remplace le méthyl-4 anisole par le méthyl-3 anisole.

La conversion du méthyl-3 anisole est de 30%.

On obtient 0,12 mmol de m-(hydroxyméthyl)anisole, 0,63 mmol de m-(acétoxyméthyl)anisole, 0,31 mmol de m-méthoxybenzaldéhyde et 0,09 mmol d'acide m-méthoxybenzoïque.

Exemple n° 23

Dans cet exemple, on met en oeuvre le méthylène dioxy-3,4 toluène.

Le catalyseur utilisé est le catalyseur contenant du palladium et de l'étain de l'exemple n° 3.

On charge dans le réacteur :

- 40 ml d'acide acétique,
- 10 ml d'eau,
- 0,98 g (10 mmol) d'acétate de potassium,
- 1,36 g (10 mmol) de méthylène dioxy-3,4 toluène,
- 1 g de catalyseur tel que préparé palladium/étain.

On agite, chauffe à 100°C pendant quatre heures, en admettant de l'oxygène à raison de 5 litres/heure.

Le réacteur est refroidi et le contenu dosé par chromatographie liquide haute performance (CLHP).

La conversion du méthylène dioxy-3,4 toluène est de 20%.

On obtient 0,2 mmol d'acétate (méthylène dioxy-3,4)benzylique, 0,4 mmol de méthylène dioxy-3,4 benzaldéhyde et 0,68 mmole d'acide (méthylène dioxy-3,4)benzoïque.

## Revendications

**1 -** Procédé d'oxydation d'un composé aromatique porteur d'un groupe alkyle oxydable de formule générale (I) :

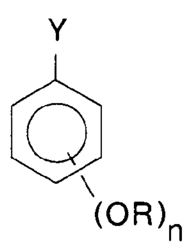

(I)

dans ladite formule (I) :

- Y représente un groupe alkyle oxydable,
- n est un nombre compris entre 1 et 5 , de préférence, égal à 1, 2 ou 3,
- R représente un radical hydrocarboné ayant de 1 à 24 atomes de carbone qui peut être un radical aliphatique saturé, linéaire ou ramifié ; un radical cycloaliphatique saturé, insaturé ou aromatique, monocyclique ou polycyclique ; un radical aliphatique saturé, linéaire ou ramifié, porteur d'un substituant cyclique,
- deux groupes R placés sur deux atomes d'oxygène portés par des atomes de carbone vicinaux du noyau aromatique peuvent former ensemble et avec les atomes d'oxygène qui les portent un cycle saturé, insaturé ou aromatique, ayant de 5 à 8 atomes,

caractérisé par le fait qu'il consiste à effectuer la réaction d'oxydation, dans l'eau ou dans un mélange comprenant de l'eau et un solvant organique, en présence d'un catalyseur solide constitué d'une phase active déposée sur support comprenant du palladium éventuellement associé à un autre élément métallique choisi parmi

l'étain, le germanium, le tellure et le cuivre.

**2 -** Procédé selon la revendication 1 caractérisé par le fait que le substrat aromatique qui est oxydé répond à la formule générale (I') :

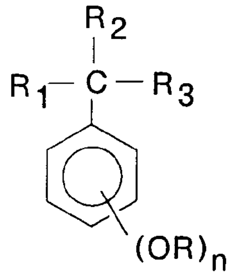

(I')

dans ladite formule (I') :

- n est un nombre égal à 1, 2 ou 3,
- $R_1$ et $R_2$ représentent un atome d'hydrogène,
- $R_3$ représente l'un des groupes suivants :
  . un atome d'hydrogène,
  . un radical alkvle avant de 1 à 12 atomes de carbone,
  . un radical phényle éventuellement substitué de formule

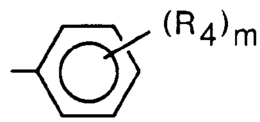

dans lequel $R_4$ représente un atome d'hydrogène, un radical alkyle ayant de 1 à 12 atomes de carbone, un radical alkoxy ayant de 1 à 10 atomes de carbone, un groupe hydroxyle ; m est un nombre égal à 0, 1, 2 ou 3,
  . un radical alkoxy ayant de 1 à 10 atomes de carbone,
  . un atome d'halogène,
  . un radical du type $R_5$ - CO - X - dans lequel $R_5$ représente un radical alkyle linéaire ou ramifié ayant de 1 à 10 atomes de carbone, un radical phényle, un radical $CF_3$ -, un radical alkoxy ayant de 1 à 10 atomes de carbone ou phénoxy et X représente un lien valentiel ou un atome d'oxygène,
- les radicaux R identiques ou différents, représentent l'un des groupes suivants :
  . un atome d'hydrogène,
  . un radical alkyle ayant de 1 à 12 atomes de carbone, de préférence 1 à 6 atomes de carbone,
  . un radical phényle éventuellement substitué,
- deux groupes R placés sur deux atomes d'oxygène portés par des atomes de carbone vicinaux du noyau aromatique peuvent former ensemble et avec les atomes d'oxygène qui les portent un cycle saturé, insaturé ou aromatique, ayant de 5 à 8 atomes.

**3 -** Procédé selon la revendication 2 caractérisé par le fait que le substrat aromatique qui est oxydé répond à la formule générale (I') dans laquelle, lorsque n est supérieur ou égal à 1, les groupes R placés sur deux atomes d'oxygène portés par des atomes de carbone vicinaux du noyau aromatique peuvent être liés entre eux pour former un cycle, par un radical alkylène ayant de 1 à 4 atomes de carbone formant ainsi un pont cétalique tel que les radicaux méthylène dioxy ou éthylène dioxy.

**4 -** Procédé selon l'une des revendications 1 à 3 caractérisé par le fait que le substrat aromatique répond à la formule générale (I) ou (I') dans laquelle le radical R représente un atome d'hydrogène ou un radical alkyle ayant de 1 à 4 atomes d'hydrogène ; de préférence le radical R représente un atome d'hydrogène.

**5 -** Procédé selon l'une des revendications 1 à 4 caractérisé par le fait que le substrat phénolique est choisi parmi :

- l'o-crésol
- le p-crésol
- le m-crésol
- l'éthyl-4 phénol

- le (méthoxyméthyl-2) phénol
- le dihydroxy-1,2 méthyl-4 benzène
- le dihydroxy-4,4' diphénylméthane

**6 -** Procédé selon l'une des revendications 1 à 5 caractérisé par le fait que le substrat phénolique est le p-crésol ou le m-crésol.

**7 -** Procédé selon l'une des revendications 1 à 6 caractérisé par le fait que le substrat aromatique est choisi parmi :

- l'o-méthylanisole,
- le p-méthylanisole,
- le m-méthylanisole,
- le dioxyméthylène-3,4 toluène,
- le p-phénoxytoluène,
- le p-hydroxyphénoxy-4 toluène.

**8 -** Procédé selon l'une des revendications 1 à 7 caractérisé par le fait que le substrat aromatique est le p-méthylanisole ou le dioxyméthylène-3,4 toluène.

**9 -** Procédé selon l'une des revendications 1 à 8 caractérisé par le fait que le solvant réactionnel est l'eau ou une solution aqueuse acide, neutre ou légèrement basique éventuellement en mélange avec un solvant organique.

**10 -** Procédé selon l'une des revendications 1 à 9 caractérisé par le fait que le solvant réactionnel comprend une solution aqueuse d'un acide minéral ou d'un sel acide tel que : l'acide borique, l'acide sulfurique, l'acétate de potassium, le carbonate de potassium, l'hydrogénosulfate de sodium, l'hydrogénosulfate de potassium ; une solution aqueuse d'un sel neutre, par exemple, le nitrate de potassium, le sulfate de potassium, le sulfate de magnésium ; une solution aqueuse d'une base minérale ou d'un sel basique tel que : l'hydroxyde de sodium, l'hydroxyde de potassium, l'hydroxyde d'ammonium, le carbonate basique de potassium.

**11 -** Procédé selon l'une des revendications 1 à 10 caractérisé par le fait que le solvant réactionnel comprend un solvant organique polaire ou apolaire, protique ou aprotique choisi parmi : les acides mono- ou polycarboxyliques ou leurs précurseurs; les alcools mono- ou polyhydroxylés ; les composés nitrés, les nitriles aliphatiques ou aromatiques; la tétraméthylène sulfone; les éther-oxydes aliphatiques, cycloaliphatiques ou aromatiques ; les esters neutres phosphoriques le carbonate d'éthylène ; les hydrocarbures aliphatiques ou aromatiques halogénés ou non.

**12 -** Procédé selon l'une des revendications 1 à 11 caractérisé par le fait que le solvant réactionnel comprend un solvant organique choisi parmi : l'acide acétique ou propionique, le méthanol, l'éthanol, le tert-butanol, l'acétonitrile, le dioxane.

**13 -** Procédé selon l'une des revendications 1 à 12 caractérisé par le fait que la quantité d"eau dans ledit mélange eau/solvant organique varie de telle sorte que le rapport pondéral entre l'eau et le solvant organique peut varier entre 0,05 et 0,95 et de préférence entre 0,20 et 0,80.

**14 -** Procédé selon l'une des revendications 1 à 13 caractérisé par le fait que le rapport molaire eau/substrat aromatique à oxyder varie entre 20 et 1000 et se situe de préférence aux environs de 100.

**15 -** Procédé selon l'une des revendications 1 à 14 caractérisé par le fait que la concentration du substrat aromatique à oxyder, dans le solvant réactionnel varie entre 0,01 mole et 5 moles de substrat aromatique par litre de solvant réactionnel et de préférence entre 0,05 et 1,0 mole par litre.

**16 -** Procédé selon l'une des revendications 1 à 15 caractérisé par le fait que le catalyseur comprend les éléments suivants : palladium, palladium-étain, palladium-germanium, palladium-cuivre, palladium-tellure, palladium-tellurecuivre, palladium-étain-cuivre et de préférence, palladium-étain ou de palladium-germanium.

**17 -** Procédé selon l'une des revendications 1 à 16 caractérisé par le fait que le catalyseur est un catalyseur à base de palladium-étain ou de palladium-germanium.

**18 -** Procédé selon l'une des revendications 1 à 17 caractérisé par le fait que les éléments métalliques du catalyseur sont déposés sur un support choisi parmi : les charbons actifs, les gels de silice, les mélanges silice-alumine, l'alumine, les argiles, la bauxite, la magnésie et la terre de diatomées.

**19 -** Procédé selon la revendications 18 caractérisé par le fait que les éléments métalliques du catalyseur sont déposés sur charbon actif.

**20 -** Procédé selon l'une des revendications 1 à 19 caractérisé par le fait que le catalyseur est pré-réduit avant d'être engagé.

**21 -** Procédé selon l'une des revendications 1 à 20 caractérisé par le fait que la phase active constituée de palladium éventuellement additionnée d'un autre élément métallique représente de 0,1 à 20 %, de préférence de 0,5 à 10 % du poids du catalyseur solide.

**22 -** Procédé selon l'une des revendications 1 à 21 caractérisé par le fait que le rapport molaire entre le palladium et les autres éléments métalliques est compris, de préférence entre 0,01 et 10, et encore plus préférentiellement entre 0,1 et 5,0.

**23 -** Procédé selon l'une des revendications 1 à 22 caractérisé par le fait que la quantité de catalyseur exprimée par le rapport molaire entre le ou les éléments métalliques et le composé aromatique porteur d'un groupe alkyle oxydable varie entre 0,01 et 0,001, de préférence entre 0,05 et 0,01.

**24 -** Procédé selon l'une des revendications 1 à 23 caractérisé par le fait que l'agent oxydant est le peroxyde d'hydrogène ou de l'oxygène moléculaire ou un gaz en contenant.

**25 -** Procédé selon l'une des revendications 1 à 24 caractérisé par le fait que la pression de réaction est comprise entre la pression atmosphérique et environ 200 bar, de préférence entre 1 et 100 bar.

**26 -** Procédé selon l'une des revendications 1 à 25 caractérisé par le fait que la température de la réaction est comprise entre 40°C et 190°C, et de préférence entre 50°C et 120°C.

Office européen des brevets

# RAPPORT DE RECHERCHE EUROPEENNE

Numero de la demande

EP 93 40 1633

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | US-A-4 915 875 (T.R. DIEPHOUSE ET AL.)<br>* colonne 3, ligne 28 - ligne 65; colonne 5, ligne 1- ligne 37; exemple 8; revendications *<br>--- | 1,2,9, 12,16, 18,19, 24-26 | C07C45/28<br>C07C45/36<br>C07C47/565<br>C07C47/575 |
| X | US-A-3 714 263 (H.A. CYBA)<br>* colonne 3, ligne 58 - ligne 75; revendications *<br>----- | 1 | |
| | | | DOMAINES TECHNIQUES RECHERCHES (Int. Cl.5 ) |
| | | | C07C |

Le présent rapport a été établi pour toutes les revendications

| Lieu de la recherche | Date d'achèvement de la recherche | Examinateur |
|---|---|---|
| LA HAYE | 08 OCTOBRE 1993 | BONNEVALLE E.I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)